# EUROPEAN PATENT APPLICATION

(11) **EP 4 101 456 A1**
(43) Date of publication of application: **14.12.2022**
(21) Application number: 20917974.6
(22) Date of filing: 06.11.2020
(51) Int. Cl.: A61K 33/44, A61P 13/12

(54) **GRAPHENE-BASED COMPOSITION FOR TREATING KIDNEY DISEASE**

(30) Priority: 05.02.2020 KR 20200013989
(71) Applicant: Biographene Inc., Seoul 06362 (KR); Seoul National University R & DB Foundation, Seoul 08826 (KR)
(72) Inventor: HONG, Byung Hee, Suwon-Si, Gyeonggi-do 16420 (KR); LEE, Jung Pyo, Seoul 03633 (KR); YANG, Seung Hee, Seoul 04593 (KR); LI, Lilin, Seoul 07060 (KR); KIM, Juhee, Seoul 08791 (KR); KIM, Donghoon, Seoul 08816 (KR); PARK, Jong Bo, Seoul 08539 (KR)
(74) Representative: Dehns
(86) International application number: PCT/KR2020/015512
(87) International publication number: WO 2021/157818

(57) **Abstract**

The present invention relates to a pharmaceutical composition for preventing or treating chronic kidney disease, the composition including graphene quantum dots as an active ingredient. Graphene quantum dots according to the present invention were observed to exhibit the effects of decreasing the expression of fibronectin, collagen 1a1, Bax, TGF-β1, Smad2/3, α-SMA, etc., which are renal fibrosis-related proteins, and increasing the expression of E-cadherin, Smad7, MnSOD, and SOD1, and thereby suppress renal fibrosis. Accordingly, it is expected that the graphene quantum dots according to the present invention will be able to be effectively used in the field of developing agents for treating or alleviating chronic kidney disease caused by renal fibrosis.

【Sequence Listing Free Text】

<110> BioGraphene Inc.

Seoul National University R&DB Foundation

<120> Composition based on graphene for treating kidney disease

<130> MPCT20-080

<150> KR 10-2020-0013989

<151> 2020-02-05

<160> 20

<170> KoPatentIn 3.0

<210> 1

<211> 20

<212> DNA

<213> Artificial Sequence

<220>

<223> Fibronectin(FN) Forward

<400> 1

cgaggtgaca gagaccacaa 20

<210> 2

<211> 20

<212> DNA

<213> Artificial Sequence

<220>

<223> Fibronectin(FN) Reverse

<400> 2

ctggagtcaa gccagacaca 20

<210> 3

<211> 20

<212> DNA

<213> Artificial Sequence

<220>

<223> Collagen 1a1(Col 1a1) Forward

<400> 3

atctcctggt gctgatggac 20

<210> 4

<211> 20

<212> DNA

<213> Artificial Sequence

<220>

<223> Collagen 1a1(Col 1a1) Reverse

<400> 4

accttgtttg ccaggttcac 20

<210> 5

<211> 20

<212> DNA

<213> Artificial Sequence

<220>

<223> alpha-smooth muscle actin(alpha-SMA) Forward

<400> 5

actgggacga catggaaaag 20

<210> 6

<211> 20

<212> DNA

<213> Artificial Sequence

<220>

<223> alpha-smooth muscle actin(alpha-SMA) Reverse

<400> 6

catctccaga gtccagcaca 20

<210> 7

<211> 24

<212> DNA

<213> Artificial Sequence

<220>

<223> TGF-beta Forward

<400> 7

caacaattcc tggcgttacc ttgg 24

<210> 8

<211> 23

<212> DNA

<213> Artificial Sequence

<220>

<223> TGF-beta Reverse

<400> 8

gaaagccctg tattccgtct cct 23

<210> 9

<211> 20

<212> DNA

<213> Artificial Sequence

<220>

<223> Bax Primer1

<400> 9

gttcccgaag taggagagga 20

<210> 10

<211> 19

<212> DNA

<213> Artificial Sequence

<220>

<223> Bax Primer2

<400> 10

ctaaagtgcc cgagctgat 19

<210> 11

<211> 24

<212> DNA

<213> Artificial Sequence

<220>

<223> Bax probe

<400> 11

aagtccagtg tccagcccat gatg 24

<210> 12

<211> 20

<212> DNA

<213> Artificial Sequence

<220>

<223> SOD1 Primer1

<400> 12

gtcctttcca gcagtcacat 20

<210> 13

<211> 21

<212> DNA

<213> Artificial Sequence

<220>

<223> SOD1 Primer2

<400> 13

ggttccacgt ccatcagtat g 21

<210> 14

<211> 24

<212> DNA

<213> Artificial Sequence

<220>

<223> SOD1 Probe

<400> 14

tccaacatgc ctctcttcat ccgc 24

<210> 15

<211> 20

<212> DNA

<213> Artificial Sequence

<220>

<223> Fibronectin(FN) Forward - human

<400> 15
   ccacccccat aaggcatagg 20

<210> 16

<211> 24

<212> DNA

<213> Artificial Sequence

<220>

<223> Fibronectin(FN) Reverse - human

<400> 16

gtaggggtca aagcacgagt catc 24

<210> 17

<211> 25

<212> DNA

<213> Artificial Sequence

<220>

<223> Collagen 1a1(Col 1a1) Forward - human

<400> 17

agggctccaa cgagatcgag atccg 25

<210> 18

<211> 24

<212> DNA

<213> Artificial Sequence

<220>

<223> Collagen 1a1(Col 1a1) Reverse - human

<400> 18

tacaggaagc agacagggcc aacg 24

<210> 19

<211> 19

<212> DNA

<213> Artificial Sequence

<220>

<223> E-cadherin(E-cad) Forward - human

<400> 19

caatgccgcc atcgcttac 19

<210> 20

<211> 24

<212> DNA

<213> Artificial Sequence

<220>

<223> E-cadherin(E-cad) Reverse - human

<400> 20

atgactcctg tgttcctgtt aatg 24

## Description

### [Technical Field]

The present invention relates to a pharmaceutical composition for treating a kidney disease, which includes graphene quantum dots (GQDs), which are graphene-based nanomaterials.

This application claims priority to and the benefit of Korean Patent Application No. 10-2020-0013989, filed on February 05, 2020, the disclosure of which is incorporated herein by reference in its entirety.

### [Background Art]

The kidney plays an important role in maintaining homeostasis in the body. It regulates the volume of a body fluid, and blood ion concentration and pH, excretes waste products such as metabolic waste, toxins, and drugs, controls blood pressure, and performs metabolic and endocrine functions. In addition, the kidney activates vitamin D to help the absorption of calcium in the small intestine and is involved in the synthesis of various hormones.

Kidney diseases refer to abnormal conditions caused by a decrease in the overall function of the kidney or the failure of the kidney to perform secretory, control, metabolic and endocrine functions normally, and classified into a chronic kidney disease and an acute kidney disease.

Chronic kidney diseases are serious diseases in which the incidence and mortality of cardiovascular diseases are 10 times higher than those of a corresponding age group, and the number of patients suffering from these diseases is increasing exponentially in recent years.

Chronic kidney diseases refer to the gradual loss of kidney function over months or years. Chronic kidney diseases can be caused by various causes, including cardiovascular disease, high blood pressure, diabetes, glomerulonephritis, polycystic kidney disease, and kidney transplant rejection.

Renal damage is associated with the release of cytokines/growth factors, such as TGF-β, an epidermal growth factor (EGF) and a platelet derived growth factor (PDGF), and an increase in TGF-β expression leads to renal fibrosis. TGF-β1 promotes fibroblast activity and induces substrate expression by interaction with a TGF-β receptor.

Glomerulosclerosis, atrophy of the tubules, and interstitial fibrosis are common signs of chronic kidney diseases. In addition, excessive accumulation and deposition of an extracellular matrix component occur. Chronic kidney diseases can eventually lead to end-stage renal failure, requiring dialysis or kidney transplantation. However, there is no treatment available to slow down the exacerbation of chronic kidney diseases.

Meanwhile, although many natural substances or chemicals for treating kidney diseases have been proposed, their current therapeutic efficacy is limited. Although many studies are being conducted on the application of nanomaterials in the life sciences as a novel therapeutic agent, no technology for treating kidney diseases by applying graphene-based nanomaterials has been suggested.

### [Disclosure]

### [Technical Problem]

The present inventors tried to develop graphene-based nano materials optimized for treating a chronic kidney disease or inhibiting renal fibrosis, and confirmed that graphene quantum dots inhibit renal fibrosis in a chronic kidney disease, and thus the present invention was completed.

Therefore, the present invention is directed to providing a pharmaceutical composition for preventing or treating a chronic kidney disease, which includes graphene quantum dots as an active ingredient.

However, technical problems to be solved in the present invention are not limited to the above-described problems, and other problems which are not described herein will be fully understood by those of ordinary skill in the art from the following description.

### [Technical Solution]

To achieve the above-described purposes, the present invention provides a pharmaceutical composition for preventing or treating a chronic kidney disease, which includes graphene quantum dots as an active ingredient.

In one embodiment of the present invention, the chronic kidney disease may be caused by renal fibrosis.

In another embodiment of the present invention, the chronic kidney disease may be one or more selected from the group consisting of end-stage kidney disease (ESKD), diabetic nephropathy (DN), IgA nephropathy (IgAN), HIV-associated nephropathy, non-diabetic chronic kidney disease, focal segmental glomerulosclerosis (FSGS), minimal change disease (MCD), and xanthine oxidase deficiency.

In still another embodiment of the present invention, the composition may reduce the expression of one or more proteins selected from the group consisting of fibronectin, collagen 1a1, Bcl-2-associated X protein (Bax), transforming growth factor-β1 (TGF-β1), Smad2/3, and α-smooth muscle actin (a-SMA).

In yet another embodiment of the present invention, the composition may increase the expression of one or more proteins selected from the group consisting of E-cadherin, Smad7, MnSOD, and SOD1.

In yet another embodiment of the present invention, the graphene quantum dots may have an average lateral size of 1 to 5 nm.

In yet another embodiment of the present invention, the graphene quantum dots may have an average height of 0.5 to 2.5 nm.

In addition, the present invention provides a method of preventing, improving or treating a chronic kidney disease, which includes administering a pharmaceutical composition including graphene quantum dots as an active ingredient into a subject.

In addition, the present invention provides a use of a pharmaceutical composition including graphene quantum dots as an active ingredient for preventing, improving or treating a chronic kidney disease.

In addition, the present invention provides a use of graphene quantum dots for producing a drug used in preventing, improving or treating a chronic kidney disease.

### [Advantageous Effects]

A pharmaceutical composition including graphene quantum dots as an active ingredient according to the present invention can inhibit renal fibrosis by reducing the expression of fibronectin, collagen 1a1, Bax, TGF-β1, Smad2/3, α-SMA, etc., which are renal fibrosis-related proteins, and increasing the expression of E-cadherin, Smad7, MnSOD, and SOD1 in mouse models with a chronic kidney disease. Therefore, it is expected that the graphene quantum dots of the present invention can be effectively used in prevention and treatment of a chronic kidney disease caused by renal fibrosis.

### [Description of Drawings]

FIG. 1A is a schematic diagram illustrating the synthesis of graphene quantum dots according to according to one embodiment of the present invention.
FIG. 1B shows a representative HR-TEM image and size distribution of graphene quantum dots according to one embodiment of the present invention.
FIG. 1C shows a representative AFM image and height distribution of graphene quantum dots according to one embodiment of the present invention.
FIG. ID shows the type of functional group confirmed by FT-IR (left) and the Raman effect confirmed through a Raman spectrometer (right) according to one embodiment of the present invention.
FIG. 2 shows the inhibitory effect on tubular atrophy of kidney tissue after the administration of graphene quantum dots in mouse models with unilateral ureteral obstruction renal fibrosis according to one embodiment of the present invention, as measured through periodic acid-Schiff (PAS) staining.
FIG. 3A shows the results of detecting the collagen deposition inhibitory effect in kidney tissue after the administration of graphene quantum dots in mouse models with unilateral ureteral obstruction renal fibrosis according to one embodiment of the present invention through Sirius Red staining, Masson's trichrome (MT) staining, and immunohistochemistry (IHC).
FIG. 3B shows the results of measuring the change in expression of related proteins during renal fibrosis after the administration of graphene quantum dots in mouse models with unilateral ureteral obstruction renal fibrosis according to one embodiment of the present invention through qRT-PCR.
FIG. 3C shows the results of measuring the change in expression of related proteins during renal fibrosis after the administration of graphene quantum dots in mouse models with unilateral ureteral obstruction renal fibrosis according to one embodiment of the present invention through western blotting.
FIGS. 4A and 4B show the results of measuring the TGF-β1/Smad signaling improvement effect after the administration of graphene quantum dots in mouse models with unilateral ureteral obstruction renal fibrosis according to one embodiment of the present invention through immunohistochemistry (IHC).
FIG. 4C shows the result of measuring the TGF-β1/Smad signaling improvement effect after the administration of graphene quantum dots in mouse models with unilateral ureteral obstruction renal fibrosis according to one embodiment of the present invention through qRT-PCR.
FIGS. 4D and 4E show the results of measuring the TGF-β1/Smad signaling improvement effect after the administration of graphene quantum dots in mouse models with unilateral ureteral obstruction renal fibrosis according to one embodiment of the present invention through western blotting.
FIGS. 5A and 5B show the results of measuring the apoptosis inhibitory effect of graphene quantum dots during renal fibrosis in mouse models with unilateral ureteral obstruction renal fibrosis according to one embodiment of the present invention through a TUNEL assay.
FIG. 5C shows the result of measuring the apoptosis inhibitory effect of graphene quantum dots during renal fibrosis in mouse models with unilateral ureteral obstruction renal fibrosis according to one embodiment of the present invention through qRT-PCR.
FIGS. 5D and 5E show the results of measuring the apoptosis inhibitory effect of graphene quantum dots during renal fibrosis in mouse models with unilateral ureteral obstruction renal fibrosis according to one embodiment of the present invention through western blotting.
FIG. 5F shows the results of measuring the renal damage protective effect of graphene quantum dots during renal fibrosis in mouse models with unilateral ureteral obstruction renal fibrosis according to one embodiment of the present invention through an immunofluorescence (IF) assay.
FIGS. 6A and 6B show the results of measuring 8-OHdG and MnSOD expression levels through immunohistochemistry (IHC) to confirm the oxidative stress inhibitory effect of graphene quantum dots during renal fibrosis in mouse models with unilateral ureteral obstruction renal fibrosis according to one embodiment of the present invention.
FIG. 6C shows the result of measuring SOD1 expression levels through qRT-PCR to confirm the oxidative stress inhibitory effect of graphene quantum dots during renal fibrosis in mouse models with unilateral ureteral obstruction renal fibrosis according to one embodiment of the present invention.
FIGS. 7A and 7B show the results of measuring the changes in kidney size and body weight by graphene quantum dots in progressive unilateral ischemia-reperfusion injury mouse models according to one embodiment of the present invention.
FIG. 8 shows the results of measuring the effect of inhibiting tubular atrophy of kidney tissue by graphene quantum dots in progressive unilateral ischemia-reperfusion injury mouse models according to one embodiment of the present invention through periodic acid-Schiff (PAS) staining.
FIG. 9 shows the results of measuring the collagen deposition inhibitory effect by graphene quantum dots in progressive unilateral ischemia-reperfusion injury mouse models according to one embodiment of the present invention through Masson's trichrome (MT) staining.
FIG. 10 shows the results of measuring the change in TGF-β1 expression by graphene quantum dots in progressive unilateral ischemia-reperfusion injury mouse models according to one embodiment of the present invention through immunohistochemistry (IHC).
FIG. 11 shows the results of measuring the change in related protein expression by graphene quantum dots during renal fibrosis in progressive unilateral ischemia-reperfusion injury mouse models according to one embodiment of the present invention through western blotting.
FIG. 12 shows the results of showing the fibrosis inhibitory effect of graphene quantum dots according to one embodiment of the present invention on recombinant TGF-β-induced human proximal tubular epithelial cells (hPTECs) through the morphological change of cells.
FIG. 13A shows the change in expression of renal fibrosis-related proteins (FN and Col 1a1) by graphene quantum dots in recombinant TGF-β-induced hPTECs according to one embodiment of the present invention through an immunofluorescence (IF) assay.
FIG. 13B shows the change in mRNA expression of renal fibrosis-related proteins (FN, Col 1a1 and E-cad) by graphene quantum dots in recombinant TGF-β-induced hPTECs according to one embodiment of the present invention through qRT-PCR.
FIGS. 14A and 14B show the apoptosis inhibitory effect by graphene quantum dots in recombinant TGF-β-induced hPTECs according to one embodiment of the present invention through Annexin V/7-amino-actinomycin D (7-AAD) flow cytometry.

### [Modes of the Invention]

The present invention provides a pharmaceutical composition for preventing or treating a chronic kidney disease, which includes graphene quantum dots as an active ingredient.

The term "graphene" used herein refers to a polycyclic aromatic molecule formed by connecting a plurality of carbon atoms by covalent bonds, which form a 6-membered ring as a basic repeating unit, but it is possible to further include 5- and/or 7-membered ring(s).

The term "graphene quantum dots (GQDs)" used herein refers to nano-sized graphene fragments.

The graphene quantum dots of the present invention may be present in a solid or liquid form. A solvate may include a non-aqueous solvent, such as ethanol, isopropanol, DMSO, acetic acid, ethanol amine, and ethyl acetate, or a solvent mixed into a crystalline lattice, such as water. A solvate containing a solvent in which water is mixed into a crystalline lattice is typically referred to as a "hydrate." The present invention includes all types of such solvates.

In the present invention, the chronic kidney disease may be caused by renal fibrosis, and may be one or more selected from the group consisting of end-stage kidney disease (ESKD), diabetic nephropathy (DN), IgA nephropathy (IgAN), HIV-associated nephropathy, non-diabetic chronic kidney disease, focal segmental glomerulosclerosis (FSGS), minimal change disease (MCD), and xanthine oxidase deficiency, but the present invention is not limited thereto.

The pharmaceutical composition according to the present invention may further include suitable carrier, excipient and diluent which are commonly used in the preparation of a pharmaceutical composition. Here, the excipient may be one or more selected from the group consisting of a diluent, a binder, a disintegrant, a lubricant, an adsorbent, a humectant, a film-coating material, and a controlled-release additive.

The pharmaceutical composition according to the present invention may be formulated in the form of a powder, a granule, a sustained-release granule, an enteric granule, a solution and a liquid, an ophthalmic solution, an elixir, an emulsion, a suspension, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a capsule, a perfusate, a plaster, a lotion, a paste, a spray, an inhalant, a patch, a sterile injection, or an external preparation such as an aerosol according to a conventional method, and the external preparation may be formulated in the form of a cream, a gel, a patch, a spray, an ointment, a plaster, a lotion, a liniment, a paste or a cataplasma.

The carrier, excipient and diluent which may be included in the pharmaceutical composition according to the present invention may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil.

The composition may be formulated with a diluent or an excipient such as a filler, a thickening agent, a binder, a wetting agent, a disintegrant, a surfactant, which are conventionally used.

As additives for a tablet, powder, granule, capsule, pill and troche, excipients such as corn starch, potato starch, wheat starch, lactose, sucrose, glucose, fructose, di-mannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose, 1928, 2208, 2906, 2910, propylene glycol, casein, calcium lactate and Primojel; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution and light anhydrous silicic acid; and lubricants such as calcium stearate, magnesium stearate, stearic acid, hydrogenated vegetable oil, talc, limestone kaolin, petrolatum, sodium stearate, cacao butter, sodium salicylate, magnesium salicylate, polyethylene glycol 4000 and, 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dileucine and light anhydrous silicic acid may be used.

As an additive for the liquid formulations according to the present invention, water, diluted hydrochloric acid, diluted sulfuric acid, sodium citrate, sucrose monostearate, polyoxyethylene sorbitol fatty acid ester (Tween ester), polyoxyethylene monoalkyl ether, lanolin ether, lanolin ester, acetic acid, hydrochloric acid, aqueous ammonia, ammonium carbonate, potassium hydroxide, sodium hydroxide, prolamine, polyvinylpyrrolidone, ethyl cellulose, or sodium carboxymethylcellulose may be used.

As the syrup according to the present invention, a sucrose solution, other sugars or sweeteners may be used, and if necessary, a fragrance, a colorant, a preservative, a stabilizer, a suspending agent, an emulsifier, or a thickening agent may be used.

As an emulsion according to the present invention, distilled water may be used, and if necessary, an emulsifier, a preservative, a stabilizer, or a fragrance may be used.

As a suspending agent according to the present invention, acacia, tragacanth, methylcellulose, carboxymethylcellulose, sodium carboxymethylcellulose, microcrystalline cellulose, sodium alginate, hydroxypropylmethylcellulose, suspending agents 1828, 2906 or 2910 may be used, and if necessary, a surfactant, a preservative, a stabilizer, a colorant, or a fragrance may be used.

For an injection according to the present invention, a solvent such as injectable sterile water, 0.9% sodium chloride for injection, Ringer's solution, dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing aid such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gum; an isotonic agent such as sodium chloride; a stabilizer such as sodium bisulfite (NaHSO3), carbon dioxide gas, sodium metabisulfite (Na2S2O3), sodium sulfite (Na2SO3), nitrogen gas (N2) or ethylenediaminetetracetic acid; an antioxidant such as sodium bisulfide 0.1%, sodium formaldehyde sulfoxylate, thiourea, disodium ethylenediaminetetraacetate or acetone sodium bisulfite; a pain-relief agent such as benzyl alcohol, chlorobutanol, procaine hydrochloride, glucose or calcium gluconate; or a suspending agent such as sodium CMC, sodium alginate, Tween 80 or aluminum monostearate, may be used.

For a suppository according to the present invention, a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium, A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or a Tegester triglyeride base (TG-95, MA, 57) may be used.

A solid formulation for oral administration may include a tablet, pill, powder, granule or capsule, and such a solid formulation may be prepared by mixing at least one of excipients, for example, starch, calcium carbonate, sucrose, lactose and gelatin, with the active ingredient. Also, in addition to simple excipients, lubricants such as magnesium stearate and talc may also be used.

As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. A formulation for parenteral administration may include a sterilized aqueous solution, a non-aqueous solvent, a suspension, an emulsion, a lyophilizing agent or a suppository. As the non-aqueous solvent or suspension, propylene glycol, polyethylene glycol, a vegetable oil such as olive oil, or an injectable ester such as ethyl oleate may be used.

The composition according to the present invention is administered at a pharmaceutically effective amount. In the present invention, the "pharmaceutically effective amount" used herein refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art.

The pharmaceutical composition of the present invention may be administered into a subject via various routes. All administration routes may be expected, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined by the type of drug as an active ingredient, in addition to various related parameters including a disease to be treated, an administration route, a patient's age, sex, and body weight and the severity of a disease.

The term "subject" used herein refers to a target in need of treatment, and more specifically, a mammal such as a human or a non-human primate, a mouse, a rat, a dog, a cat, a horse, or a cow.

In the present invention, the "administration" refers to providing the composition of the present invention to a subject by any suitable method.

The "prevention" used herein refers to all actions of inhibiting or delaying the onset of a target disease, the "treatment" used herein refers to all actions involved in alleviating or beneficially changing symptoms of a target disease and its metabolic abnormalities by the administration of a pharmaceutical composition according to the present invention, and the "improvement" refers to all types of actions that at least reduce parameters related to a target disease, for example, the degree of a symptom, by the administration of a composition according to the present invention.

In the pharmaceutical composition according to the present invention, a therapeutically effective amount is not particularly limited, but may be, preferably 5 to 50 mg/kg, more preferably 10 to 40 mg/kg, and most preferably 20 to 30 mg/kg.

In the present invention, the composition may prevent or treat a chronic kidney disease caused by renal fibrosis by reducing the expression of one or more proteins selected from the group consisting of fibronectin, collagen 1a1, Bcl-2-associated X protein (Bax), transforming growth factor-β1 (TGF-β1), Smad2/3, and α-smooth muscle actin (a-SMA).

In the present invention, the composition may prevent or treat a chronic kidney disease caused by renal fibrosis by increasing the expression of one or more proteins selected from the group consisting of E-cadherin, Smad7, MnSOD, and SOD1.

In the present invention, the "fibronectin" is an extracellular matrix glycoprotein that binds to integrin, which is a transmembrane receptor protein, and is known to be mainly expressed by the activity of fibroblasts and mainly expressed at the beginning of the fibrosis process in various organs.

The term "a-smooth muscle actin (a-SMA)" used herein is known as α-actin-2, SMactin, or ASMA, and it is a protein expressed by the ACTA2 gene, and a molecular marker exhibiting the activation of a myofibroblast in the fibrosis of all types of organs.

In the present invention, the "E-cadherin" is one of the cell adhesion molecules, and plays an important role in forming an adherent junction for attaching cells. It has a length of approximately 720 to 750 amino acids, consists of a small cytoplasmic region, a transmembrane region, and a large extracellular region, and is lost by TGF-β1 inducing fibrosis. It is known that the loss of E-cadherin cause fibrosis.

The "transforming growth factor-β1 (TGF-β1)" used herein is a multifunctional cytokine belonging to the transformation growth factor superfamily that includes various isoforms, and is known to be a main factor inducing the fibrotic form of most chronic kidney diseases.

The "Smad2/3" used herein is a main signal transducer for receptors of the TGF-β superfamily, and constitutes a structurally similar protein group.

The "Smad7" used herein inhibits TGF-β signaling by preventing the formation of a Smad2/Smad4 complex initiating the TGF-β signaling. It blocks the binding, phosphorylation and activation of Smad2 by interaction with an activated TGF-β type I receptor.

The "8-hydroxy-2'-deoxyguanosine (8-OHdG)" used herein is an oxidized derivative of deoxyguanosine produced by DNA oxidation, and the expression level of 8-OHdG in cells indicates an oxidative stress level.

The "manganese superoxide dismutase (MnSOD)" and "superoxide dismutase1 (SOD1)" used herein are enzymes that convert a superoxide into oxygen and hydrogen peroxide, and is known to relieve oxidative stress in cells.

The term "expression" refers to the process of producing a polypeptide from a structural gene. This process includes gene transcription into mRNA, and the translation of the mRNA into a polypeptide(s).

In the present invention, as an analysis method for measuring an mRNA expression level, a method known in the art, for example, polymerase chain reaction (PCR), reverse transcription polymerase chain reaction (RT-PCR), competitive RTPCR, real-time PCR (quantitative PCR), RNase protection assay (RPA), Northern blotting, or DNA chip, may be used. According to one embodiment of the present invention, the mRNA expression level may be measured using real-time PCR (quantitative PCR), but the present invention is not limited thereto.

In the present invention, as an analysis method for measuring a protein expression level, a method known in the art, for example, Western blotting, enzyme linked immunosorbent assay (ELISA), radioimmunoassay (RIA), radioimmunodiffusion, Ouchterlony immune diffusion, rocket immunoelectrophoresis, immunohistochemistry, immunoprecipitation assay, complement fixation assay, fluorescence activated cell sorter (FACS), or a protein chip, may be used, but the present invention is not limited thereto.

In the pharmaceutical composition according to the present invention, the graphene quantum dots may have an average lateral size of 1 to 5 nm, and an average height of 0.5 to 2.5 nm.

The present invention also provides a method of preventing, improving or treating a chronic kidney disease, which includes administering a pharmaceutical composition including graphene quantum dots as an active ingredient into a subject.

The present invention also includes a use of a pharmaceutical composition including graphene quantum dots as an active ingredient for preventing, improving or treating a chronic kidney disease.

In addition, the present invention provides a use of graphene quantum dots for producing a drug used in preventing, improving or treating a chronic kidney disease.

In one embodiment of the present invention, graphene quantum dots were prepared, and their characteristics were analyzed by identifying the size of graphene quantum dots and the type of functional group (see Example 1).

In one experimental example of the present invention, by analyzing effects of inhibiting tubular atrophy, collagen deposition, changing the expression of renal fibrosis-related proteins, improving TGF-β1/Smad signaling, inhibiting apoptosis, and inhibiting oxidative stress in unilateral ureteral obstruction models, the renal fibrosis inhibitory effect of the graphene quantum dots of the present invention was confirmed (see Experimental Example 1).

In another experimental example of the present invention, by analyzing the changes in kidney size and weight, inhibition of tubular atrophy, inhibition of collagen deposition, inhibition of TGF-β1 expression, and the change in expression of renal fibrosis-related proteins in unilateral ischemia-reperfusion injury mouse models, and the renal fibrosis inhibitory effect of the graphene quantum dots of the present invention was confirmed (see Experimental Example 2).

In still another experimental example of the present invention, by analyzing effects of the morphological change in cells, the change in expression of renal fibrosis-related proteins, and inhibition of apoptosis of human proximal tubular epithelial cells, the renal fibrosis inhibitory effect of the graphene quantum dots of the present invention was confirmed (see Experimental Example 3).

Hereinafter, to help in understanding the present invention, exemplary examples will be suggested. However, the following examples are merely provided to more easily understand the present invention, and not to limit the present invention.

### Example 1. Preparation of graphene quantum dots (GODs)

### 1-1. Preparation of GQDs

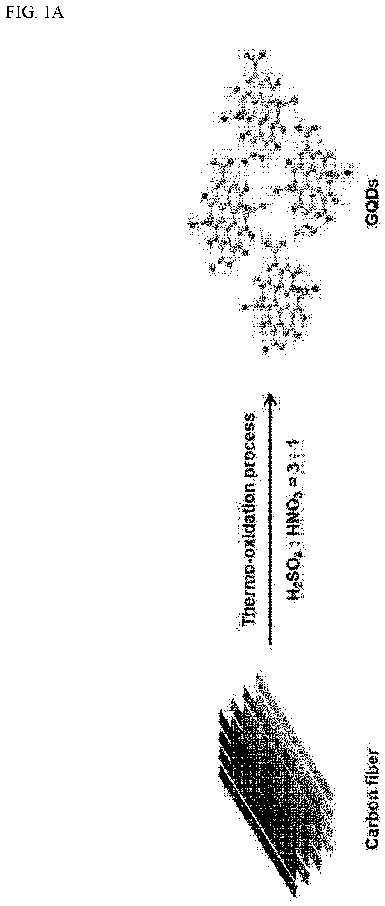

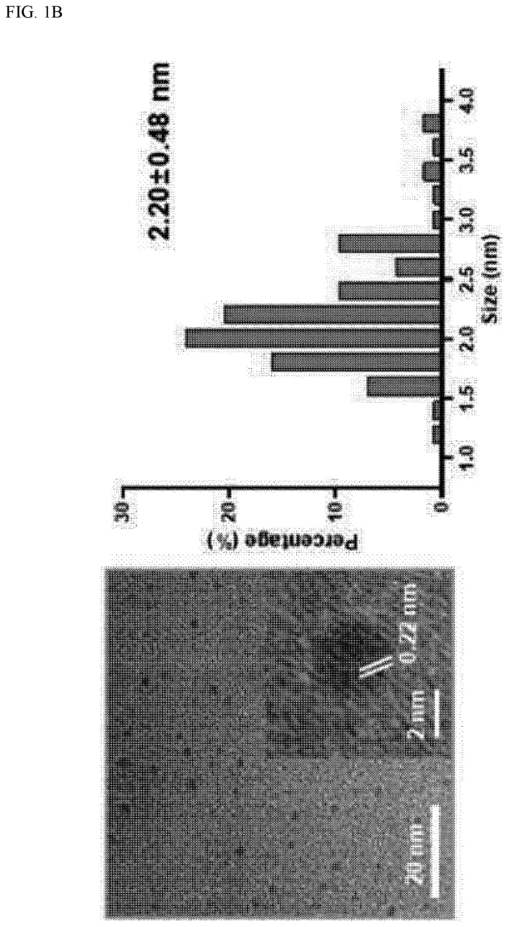

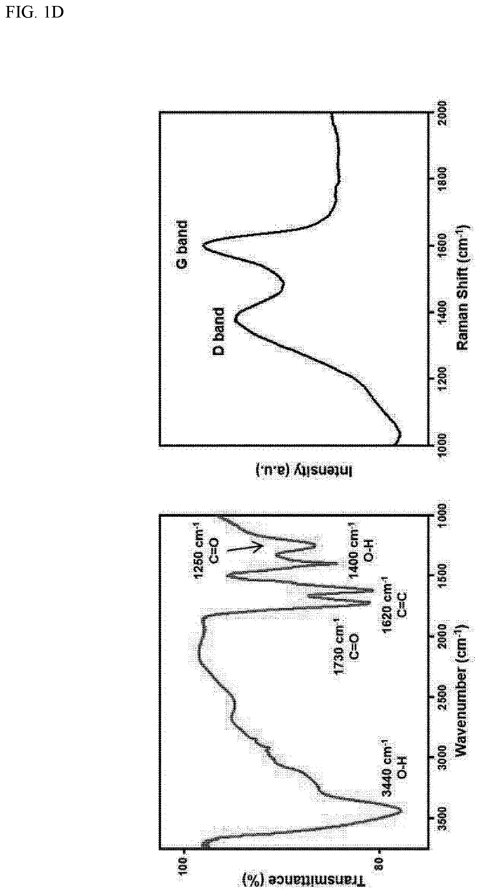

Carbon fiber was put into a strong acid solution in which sulfuric acid and nitric acid were mixed in a 3:1 ratio, and then heated at 80 °C for 24 hours (thermo-oxidation process). After the solution was diluted, the resulting solution was dialyzed using a regenerated nitrocellulose membrane (Cat#: 06-680-2G; MWCO 1,000 daltons; Fisher Scientific) to remove an acid, vacuum-filtered using a porous inorganic membrane filter (Cat#: 6809-5002; Whatman-Anodisc 47; GE Healthcare), and then freeze-dried, thereby preparing GQDs in powder form. The GQD powder was redispersed in distilled water for use in an experiment (FIG. 1A).

### 1-2. Analysis of characteristics of GQDs

To measure the average form of GQDs, a lateral size was measured through high-resolution transmission electron microscopy (HR-TEM), a height was measured through atomic force microscopy (AFM), and the type of functional group was identified through Fourier-transform infrared spectroscopy (FT-IR). In addition, the Raman effect was confirmed through Raman spectroscopy.

Before the use of HR-TEM, a 300-mesh copper TEM grid having lacey carbon (01890-F, TedPella, USA) was coated with graphene. The diluted graphene quantum dot solution was dropped on the grid and dried in the air. The image of the graphene quantum dots was confirmed using HR-TEM (JEM-3010, JEOL Ltd, Japan) and the Gatan Digital Camera (MSC-794). The lateral size of the graphene quantum dot was measured by Image J.

AFM (Park Systems, Suwon, Korea) was used to analyze a sample, which was prepared by dropping the graphene quantum dot solution on a 1x1 Si wafer and drying the wafer, in a contact mode.

For FT-IR, a graphene quantum dot sample was prepared by a common KBr pellet method, and its spectrum was detected using an FT-IR spectrometer (Nicolet 6700, Thermo Scientific, USA).

The Raman effect was measured by preparing a sample by dropping a graphene quantum dot solution on a 1x1 Si wafer and drying the wafer, and then detecting a spectrum using a 514-nm laser Raman spectrometer (Micro-Raman spectrometer, Renishaw, UK).

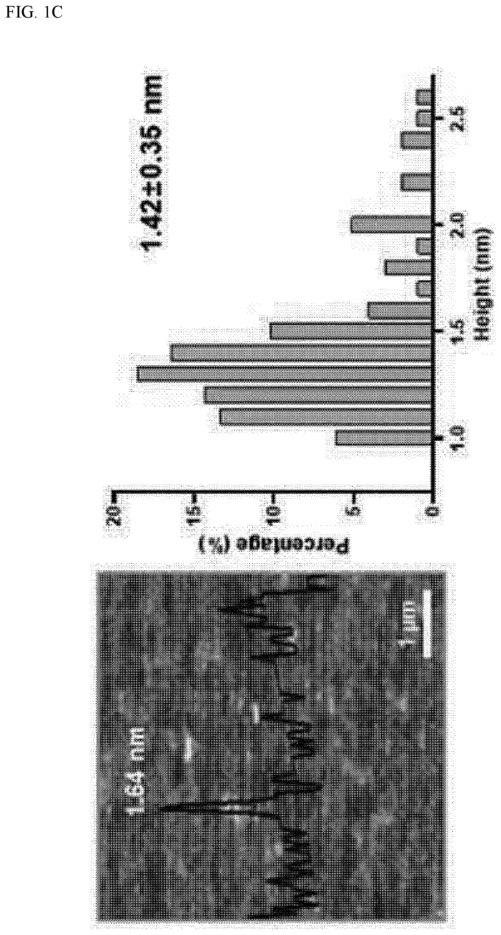

As a result, the lateral size of the graphene quantum dot was 2.20 nm (FIG. IB), and the height thereof was 1.42 nm (FIG. 1C). The wave numbers corresponding to stretching of the carboxyl group (C=O) and the hydroxyl group (O-H) at an edge were 1730 cm⁻¹ and 3440 cm⁻¹, respectively, and the wave number corresponding to sp² C=C stretching corresponding to the graphene region was observed at 1620 cm⁻¹ (FIG. ID, left). As a result of Raman shift analysis, the D band (1400 cm⁻¹) and G band (1600 cm⁻¹) for graphene were detected (FIG. ID, right).

### Experimental Example 1. Effect of renal tissue fibrosis inhibitory effect in unilateral ureteral obstruction (UUO) models

### 1-1. Preparation of animal model

7-week-old male mice (C57BL/6 mice) were anesthetized by intraperitoneal administration of Zoletil (30 mg/kg) and Rompun (10 mg/kg), and after finding the ureter by exposing the kidney by making an incision on the left side of each mouse, the proximal part of the ureter was tied with 4-0 silk at intervals of 1 to 2 mm, and an incision was made therebetween, thereby obtaining a chronic kidney disease animal test model, that is, a UUO mouse model. The graphene quantum dots according to Example 1 were administered to the caudal vein of each mouse at a dose of 20 mg/kg/day one day before surgery and on days 2 and 6 after surgery, and then on day 10 after the UUO procedure, the mouse was sacrificed to extract the kidneys, and the induction of renal fibrosis was observed.

The mice were classified into groups: Sham: normal control/vehicle administration; GQDs: normal control/GQD administration; UUO: UUO experimental group/vehicle administration; UUO + GQDs: UUO experimental group/GQD administration. A control was administered a vehicle (n=7 per group)

### 1-2. Analysis of tubular atrophy inhibitory effect

To confirm the difference in the degree of renal fibrosis between a control and an experimental group, histological changes in the tubules were observed through periodic acid-Schiff (PAS) staining.

The extracted kidney was fixed in 4% paraformaldehyde at 4 °C for 24 hours and embedded in paraffin. After dehydration, a paraffin block was cut into 4-µm sections. The sections were put into an oven at 60 °C for 1 hour, and deparaffinized with ethanol and paraffin, and then PAS staining was performed according to the protocol of a periodic acid-Schiff Stain Kit (ScyTek; Logan, Utah, USA).

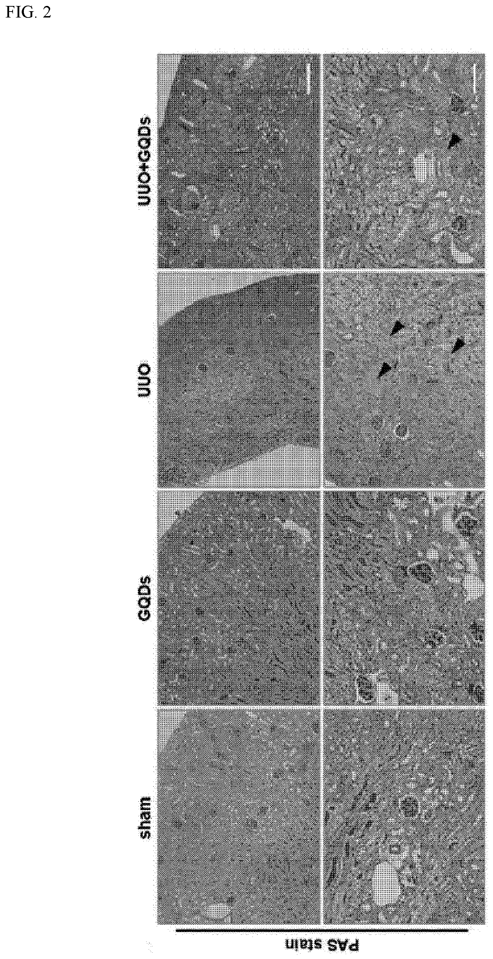

As a result, from PAS staining, clear tubular atrophy was observed in the UUO experimental group, but a relatively intact structure was found in the UUO + GQD group (FIG. 2).

### 1-3. Analysis of collagen deposition inhibitory effect

To confirm the difference in the degree of renal fibrosis between a control and an experimental group, the degree of collagen deposition was confirmed by performing Sirius Red staining, Masson's trichrome (MT) staining and immunohistochemistry (IHC) of collagen 1a1 (Col 1a1).

Renal tissue samples were prepared in the same manner as in Experimental Example 1-2, and Sirius Red (ScyTekv; Logan, Utah, USA) staining was performed with a Picro-Sirius Red Stain Kit (For Collagen), and MT staining was performed according to the protocol of a Trichrome Stain Kit (Modified Masson's).

For immunohistochemistry assay, kidney tissue samples were also prepared as described above, 3% hydrogen peroxide (Duksan; Ansan, Gyeonggi-do, Korea) and 10% goat serum (RD, Minneapolis, MN, USA) were used to block endogenous peroxide enzymes and 10 non-specific expressions in the tissue sections. Afterward, Col 1a1 expression was evaluated by incubation overnight at 4 °C with the primary antibody, which is a Col 1a1 (Abcam; ab21286) antibody.

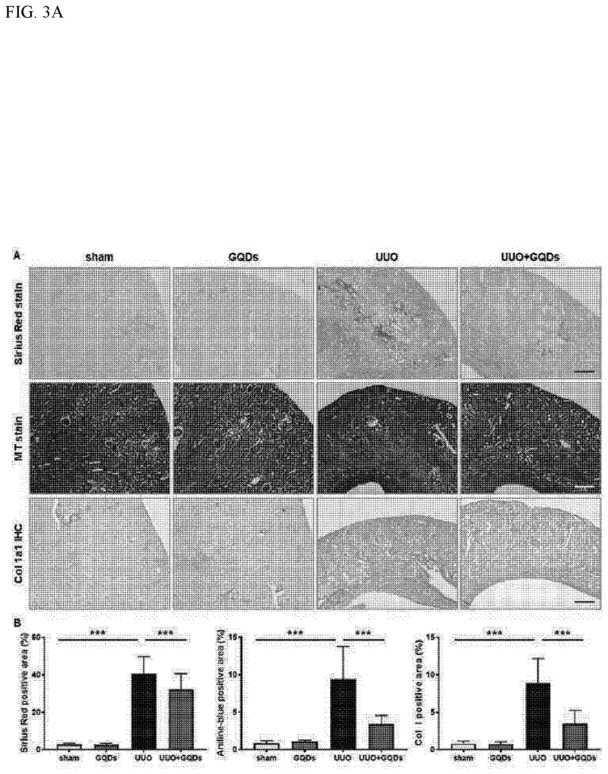

As a result, in the UUO + GQD group, compared to the UUO experimental group, the positive area of Sirius Red staining was reduced by 20%, and it was confirmed that the positive area was reduced by approximately 60% according to MT staining and Col 1a1 IHC, confirming that collagen deposition was reduced by the graphene quantum dots (FIG. 3A).

### 1-4. Analysis of change in expression of renal fibrosis-related protein

To analyze the changes in related protein expression during UUO-induced renal fibrosis, quantitative real-time (qRT)-PCR and western blotting were performed.

For qRT-PCR, total RNA was extracted from cells and tissues using a Trizol reagent (Bioline; Luckenwalde, Germany) according to the manufacturer's instruction. The quality and concentration of the RNA extract were measured by 260/280 nm spectrophotometry. Subsequently, each sample at the same concentration was reverse-transcribed into cDNA using a synthesis kit and amplification equipment (C1000^{™}, Thermal Cycle, Bio-Rad). The same concentration of cDNA was added to a TaqMan probe or SYBR Green primer together with a PCR master mixture, and then measured using LightCycler480 instrument II (Roche; Pleasanton, CA, USA). The relative expression level of each gene was calculated using a comparative threshold (CT) cycle (2^{-ΔΔCT}). The TaqMan probe or SYBR Green primer sequences used in this experiment are shown in Table 1 below.

**[Table 1]**

| **Primer** | **Sequence** | **Species** | **SEQ. ID NO:** |
|---|---|---|---|
| Fibronectin (FN) Forward | CGA GGT GAC AGA GAC CAC AA | mouse | 1 |
| Fibronectin (FN) Reverse | CTG GAG TCA AGC CAG ACA CA | mouse | 2 |
| Collagen 1a1(Col 1a1) Forward | ATC TCC TGG TGC TGA TGG AC | mouse | 3 |
| Collagen 1a1(Col 1a1) Reverse | ACC TTG TTT GCC AGG TTC AC | mouse | 4 |
| α-smooth muscle actin(α-SMA) Forward | ACT GGG ACG ACA TGG AAA AG | mouse | 5 |
| α-smooth muscle actin(α-SMA) Reverse | CAT CTC CAG AGT CCA GCA CA | mouse | 6 |

In Western blotting, the proteins extracted from cells and tissues were prepared as proteins for electrophoresis, followed by electrophoresis and membrane transfer. After transfer, the membrane was incubated at least 1 hour in a blocking buffer, and then incubated overnight at 4 °C together with a primary antibody FN (Santa Cruz, sc-8422), Col 1a1 (Abcam, ab21286), E-cad (Abcam, ab11512), α-SMA (Abcam, ab5694), and GAPDH (Cell Signaling Technology, 2118). Subsequently, the membrane was washed with a TBST buffer three times for 10 minutes. And then, the membrane was incubated in a blocking buffer containing an anti-rabbit IgG, HRP-linked antibody or an anti-mouse IgG, HRP-linked antibody (both, Cell Signaling Technology; Danvers, MA, USA). After washing, the membrane was developed using a western Bright ECL kit (Advansta; San Jose, CA, USA) in a Luminescent Image Analyzer LAS4000 system (Fuji Film; Dusseldorf, Germany).

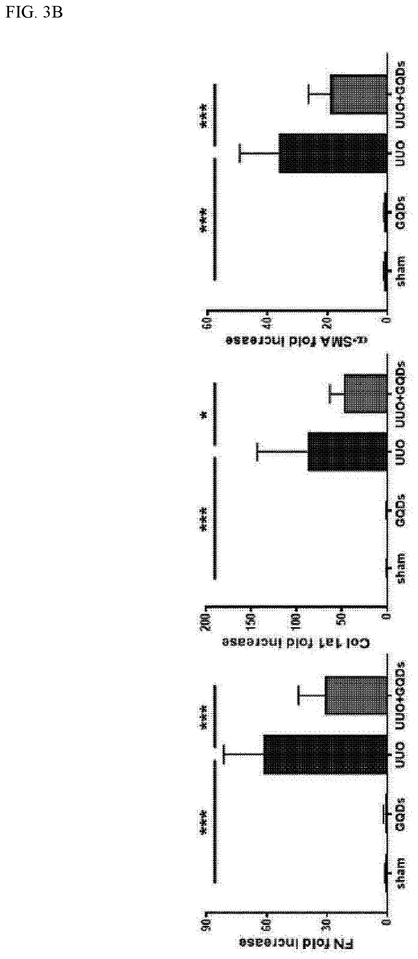

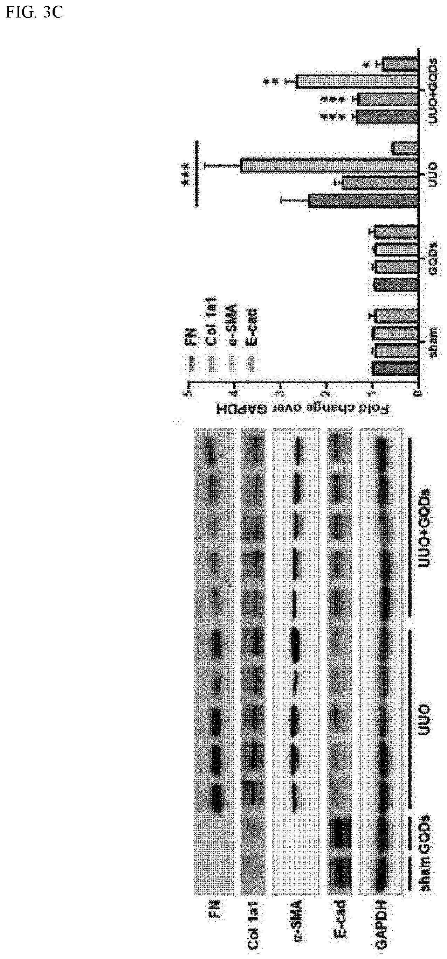

As a result, during renal fibrosis, mRNA levels of extracellular matrix (ECM) proteins such as fibronectin (FN), collagen 1a1 (Col 1a1), and α-smooth muscle actin (a-SMA) significantly increased. However, it was confirmed that FN, Col 1a1 and α-SMA significantly decreased in the presence of graphene quantum dots (FIG. 3B). Likewise, in western blotting analysis, the expression of FN, Col 1a1 and α-SMA was reduced. Meanwhile, the expression level of E-cadherin was increased by graphene quantum dots, suggesting that the inhibition of the epithelial-mesenchymal transition (EMT) disturbs cell-cell attachment less (FIG. 3C).

### 1-5. Analysis of TGF-β1/Smad signaling improvement effect

Since the TGF-β1/Smad signaling pathway is known to be mainly associated with renal fibrosis, immunohistochemistry (IHC), qRT-PCR and western blotting were performed to confirm an effect of graphene quantum dots on TGF-β1/Smad signaling.

Immunohistochemistry was performed in the same manner as in Experimental Example 1-3, and TGF-β1 (Abcam, ab92486) was used as a primary antibody.

qRT-PCR was performed in the same manner as in Experimental Example 1-4, and primer sequences are shown in Table 2 below.

**[Table 2]**

| **Primer** | **Sequence** | **Species** | **SEQ ID NO:** |
|---|---|---|---|
| TGF-β Forward | CAA TTC CTG GCG TTA CCT TGG | mouse | 7 |
| TGF-β Reverse | GAA AGC CCT GTA TTC CGT CTC CT | mouse | 8 |

Western blotting was also performed in the same manner as in Experimental Example 1-4, and TGF-β1 (Abcam, ab92486), p-smad2/3 (Abcam, ab63399), smad2/3 (Cell Signaling Technology, 8685), Smad7 (Abcam, ab216428) and GAPDH (Cell Signaling Technology, 2118) were used as primary antibodies.

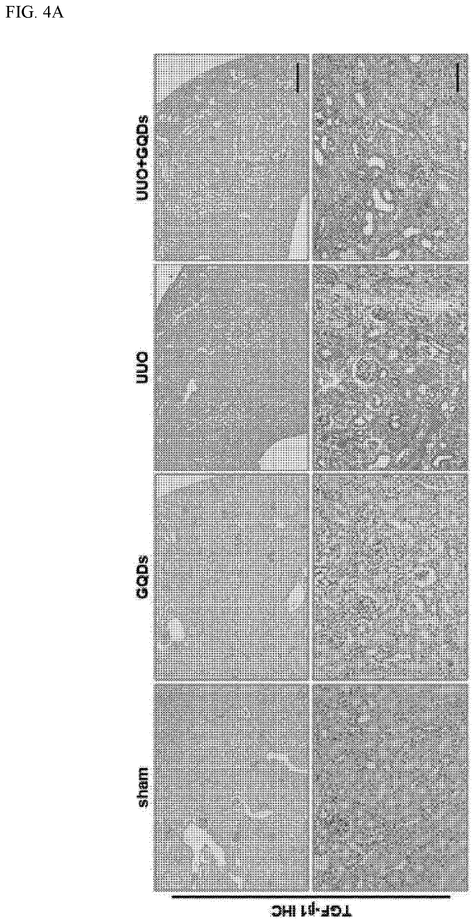

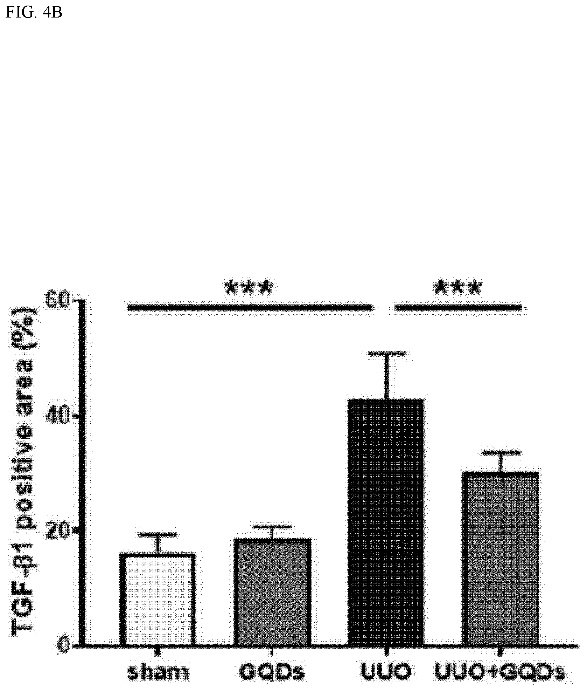

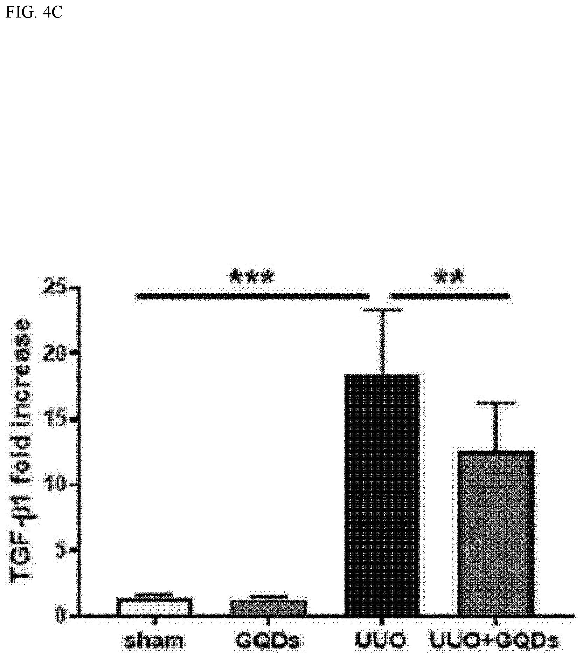

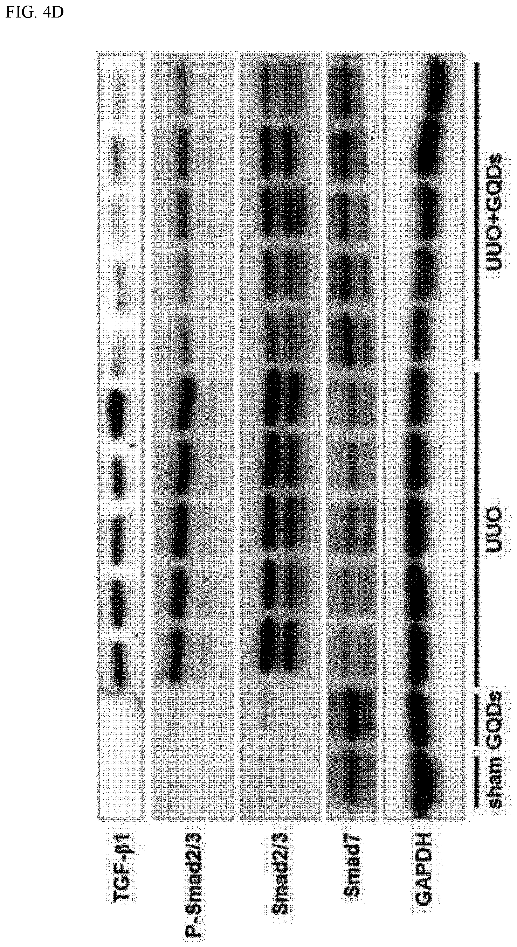

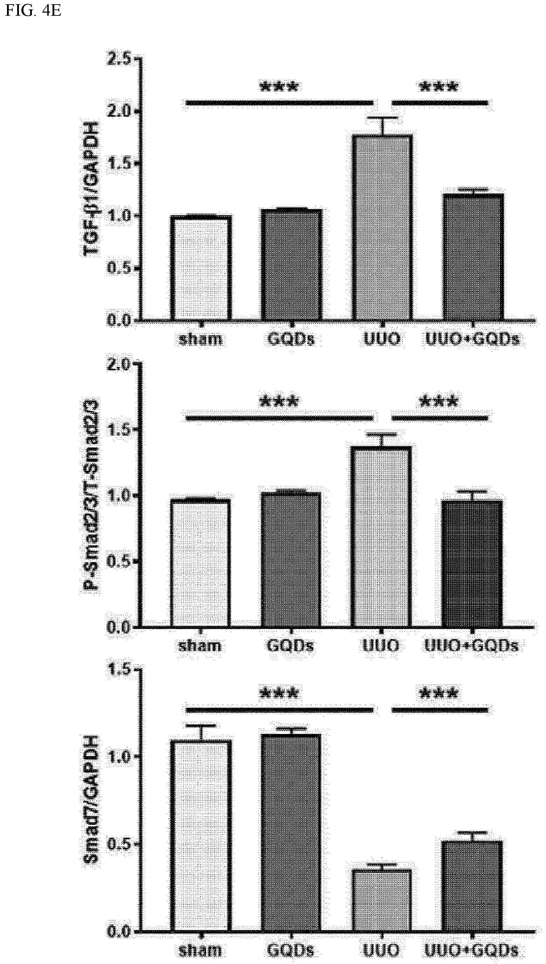

As a result, in the immunohistochemistry analysis, 10 days after UUO induction, TGF-β1 expression in the obstructed kidney was significantly higher than the control, and the positively-stained area of TGF-β1 was reduced by 30% in the graphene quantum dot-administered group (FIGS. 4A and 4B). The graphene quantum dots also reduced the mRNA levels of TGF-β1 (FIG. 4C). As a result of the western blotting experiment, changes were also observed in the downstream proteins of the TGF-β1/Smad signaling pathway. Due to the administration of graphene quantum dots, the level of p-Smad2/3 promoting the production of ECM proteins was reduced by 30%. On the other hand, Smad7 interfering with the activation of a Smad2/3 complex was 1.5-fold increased by the administration of graphene quantum dots (FIGS. 4D and 4E). Therefore, it was confirmed that graphene quantum dots inhibited excessive formation of ECM proteins through overall regulation of the TGF-β1/Smad signaling pathway.

### 1-6. Analysis of apoptosis inhibitory effect and renal damage protective effect during renal fibrosis

To confirm the apoptosis inhibitory effect of graphene quantum dots, TUNEL assay, qRT-PCR, western blotting and immunofluorescence (IF) assay were performed.

The TUNEL assay was used to detect an apoptosis marker from a paraffin-embedded kidney tissue section using an apoptosis detection kit (ApopTag^{®} Plus Peroxidase *In Situ* Apoptosis Kit, Merck Millipore; Billerica, MA, USA). The paraffin section was deparaffinized, and incubated with proteinase K (Dako; Carpinteria, CA, USA), and then incubated at 37 °C for 30 minutes together with an enzyme terminal deoxynucleotide transferase (TdT, used by diluting in reaction buffer 37 °C for 1 hour). In addition, the resulting product was conjugated with anti-digoxigenin (Roche; Mannheim, Germany). The nucleus was counterstained with DAPI. The degree of apoptosis was assessed in a blinded manner by averaging the total number of apoptotic cells in 10 randomly selected regions observed (×200) in each slice.

qRT-PCR was performed in the same manner as in Experimental Example 1-4, and primer and probe sequences are shown in Table 3 below.

**[Table 3]**

| **Primer/Probe** | **Sequence** | **Species** | **SEQ. ID. NO:** |
|---|---|---|---|
| Bax Primer1 | GTTCCCGAAGTAGGAGAGGA | mouse | 9 |
| Bax Primer2 | CTAAAGTGCCCGAGCTGAT | mouse | 10 |
| Bax Probe | /56- FAM/AAGTCCAGT/ZEN/GTCCAGCCCATGATG/3IABkFQ/1 | mouse | 11 |

Western blotting was performed in the same manner as in Experimental Example 1-4, and Bax (Cell Signaling Technology, 2772), Bcl2 (Thermo Fisher, MA5-11757) and GAPDH (Cell Signaling Technology, 2118) were used as primary antibodies.

For IF analysis, after deparaffinization, rehydration and antigen recovery, kidney tissue sections were incubated overnight at 4 °C with primary antibodies such as AQP1 (Abcam, ab15080) and NGAL (Santa Cruz, sc-515876) in antibody dilution buffer. Afterward, sufficient amounts of goat-anti-rabbit Alexa Fluor488 and goat-anti-mouse Alexa Fluor555 (both, Invitrogen; OR, USA) as secondary antibodies were added to cover the sections, followed by incubation in a dark place for 30 minutes at room temperature. Subsequently, a mounting solution containing 4', 6-diamidino-2-phenylindole (DAPI; Thermo Fisher Scientific; Waltham, MA, USA) was used to mount slides and for counterstaining. Confocal microscopy (Leica TCS SP8 STED CW; Wetzlar, Germany) was used to obtain high-quality fluorescence images.

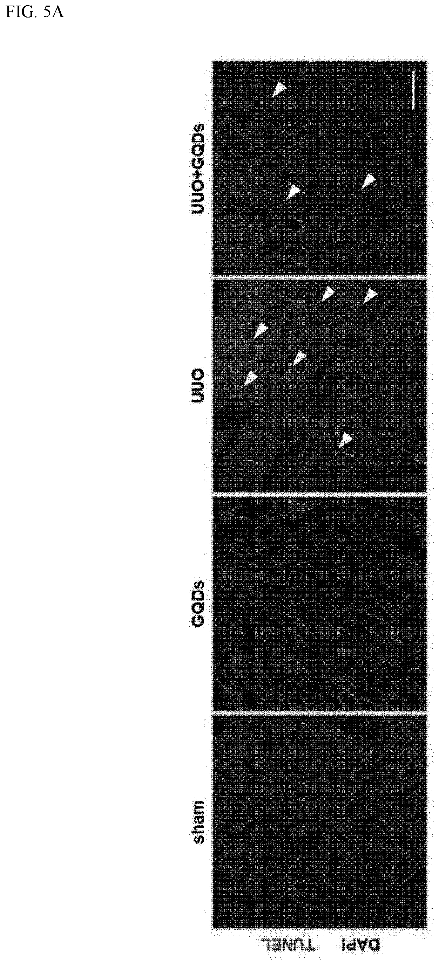

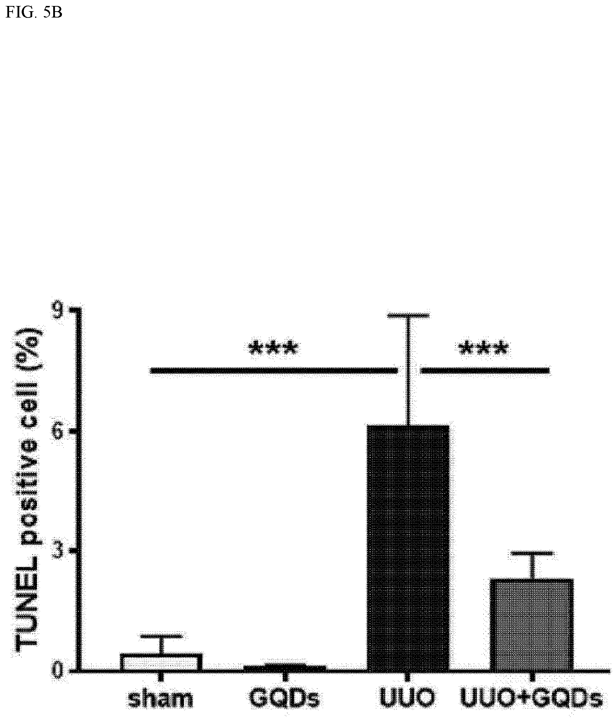

As a result, as shown from the stained images and quantified results, the presence of graphene quantum dots significantly decreased the number of TUNEL-positive apoptotic cells (FIGS. 5A and 5B).

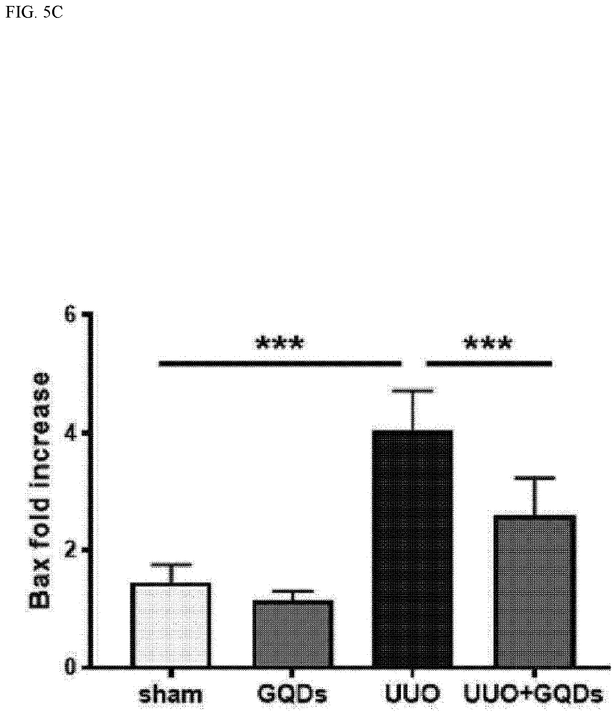

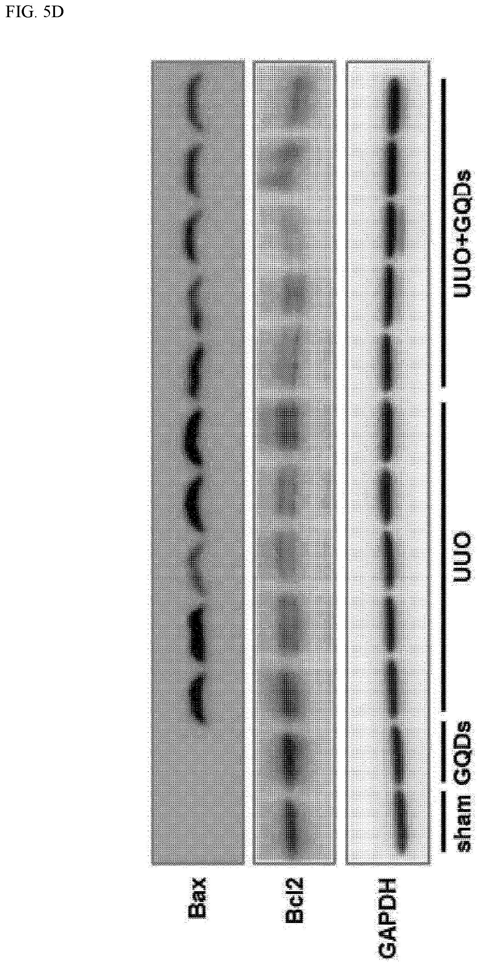

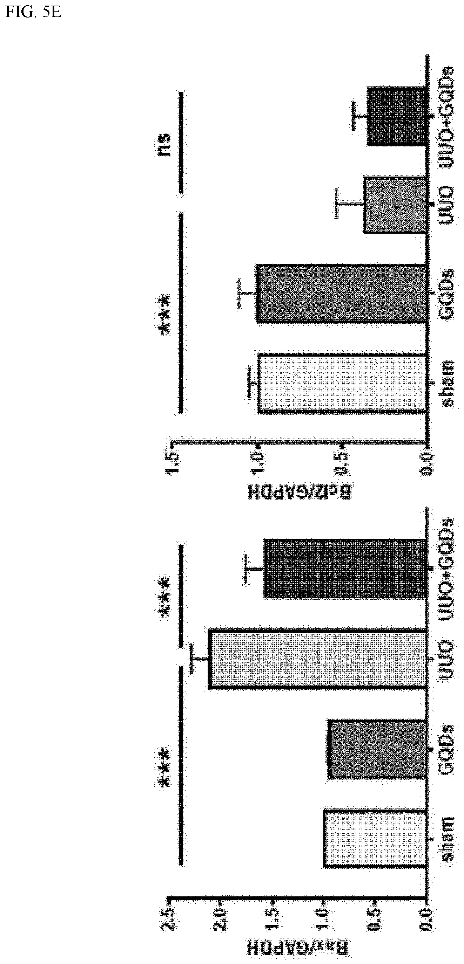

Likewise, graphene quantum dots reduced Bcl2-associated X protein (Bax) at both mRNA and protein levels (FIGS. 5C, 5D and 5E). However, there was no significant difference in Bcl2 (FIGS. 5D and 5E), indicating that graphene quantum dots inhibit apoptosis by inhibiting the production of a proapoptotic factor rather than restoring an anti-apoptotic protein.

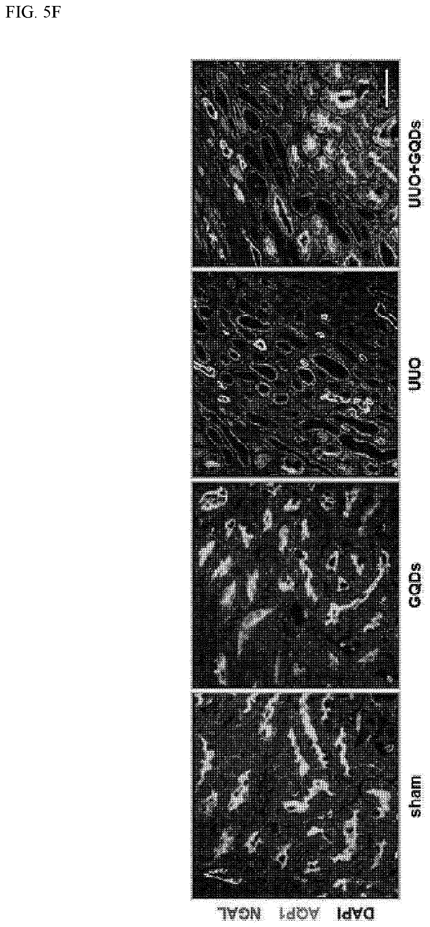

To verify the effects of graphene quantum dots on the preservation of tubular cells, the degree of renal damage was measured by immunofluorescence (IF) staining of neutrophil gelatinase-associated lipocalin (NGAL) and aquaporin (AQP1). NGAL is a novel biomarker, which may be considered as a parameter for the progression of an acute kidney disease to a chronic kidney disease. AQP1 is located in the proximal tubule and serves as a channel. According to the experimental result, a significant increase in NGAL and a loss of AQP1 were observed in the UUO group, but it was confirmed that the changes in NGAL and AQP1 were significantly weakened by graphene quantum dots (FIG. 5F), suggesting a protective effect of graphene quantum dot against proximal tubular damage.

### 1-7. Analysis of oxidative stress inhibitory effect

Since the accumulation of reactive oxygen species (ROS) is known to cause oxidative stress and exacerbate fibrosis, to identify the effect of graphene quantum dots on ROS-induced oxidative stress, the expression of 8-OHdG and MnSOD was confirmed by immunohistochemistry (IHC), and SOD1 expression was confirmed by qRT-PCR.

Immunohistochemistry was performed in the same manner as in Experimental Example 1-3, and 8OHdG (JaICA; M0G0209) and MnSOD (Abcam; Ab31533) were used as primary antibodies. qRT-PCR was performed in the same manner as in Experimental Example 1-4, and primer and probe sequences are shown in Table 5 below.

**[Table 4]**

| **Primer/Probe** | **Sequence** | **Species** | **SEQ ID NO:** |
|---|---|---|---|
| SOD1 Primer1 | GTCCTTTCCAGCAGTCACAT | mouse | 12 |
| SOD1 Primer2 | GGTTCCACGTCCATCAGTATG | mouse | 13 |
| SOD1 Probe | /56- FAM/TCCAACATG/ZEN/CCTCTCTTCATCCGC/3IABkFQ/ ; n | nouse | 14 |

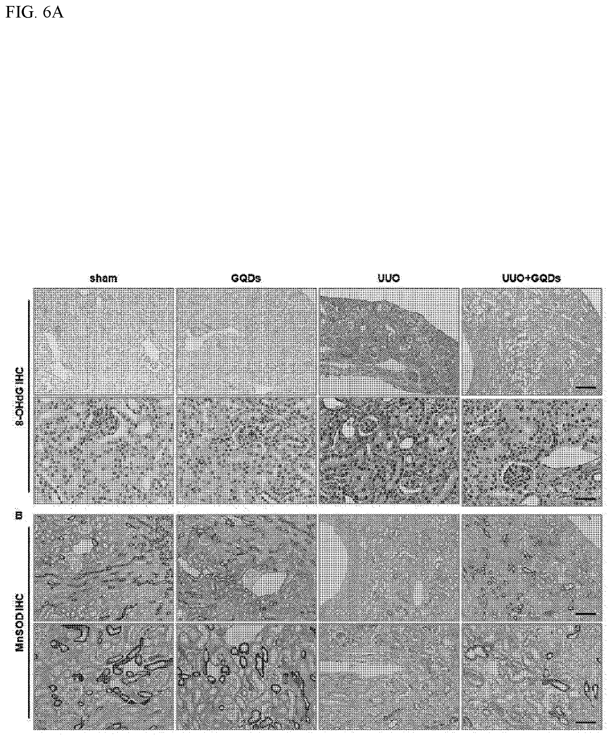

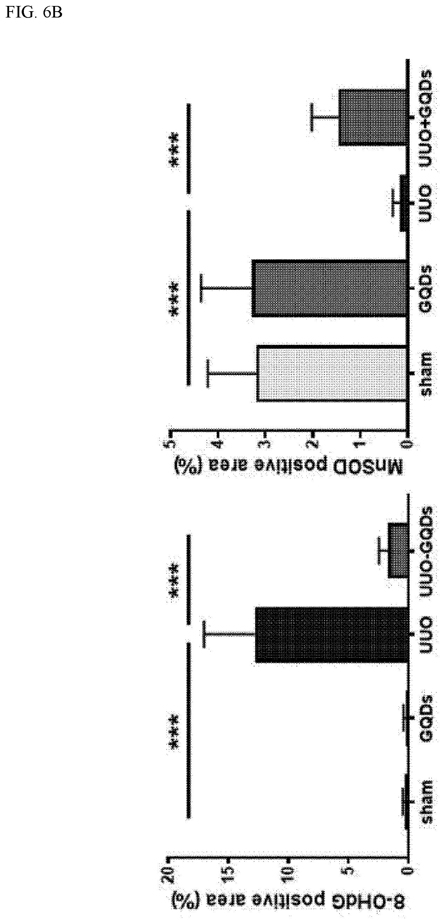

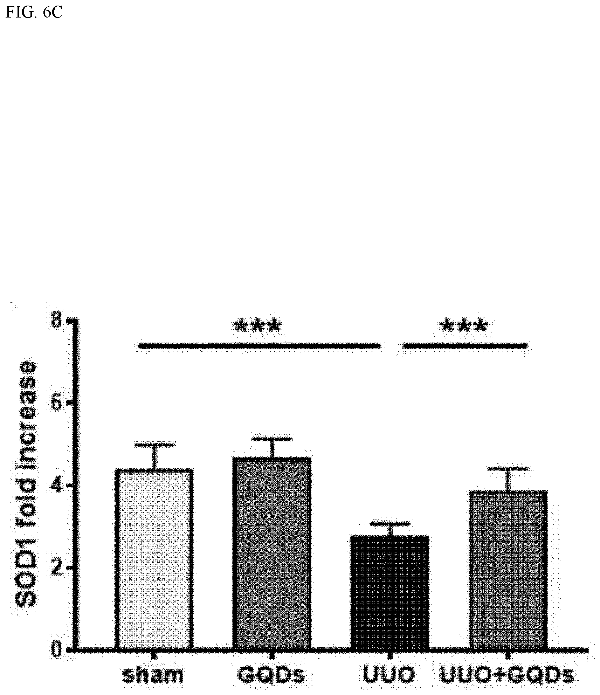

As a result, compared to the UUO experimental group, in the UUO + GOD group, the 8-OHdG-positive area was reduced by 87%, whereas it was confirmed that the MnSOD-positive area increased 9-fold (FIGS. 6A and 6B), and it was confirmed that the mRNA expression of SOD1 is restored to a normal level (FIG. 6C). The above results suggest that graphene quantum dots cannot only scavenge ROS, but also inhibit oxidative stress by regulating the balance between an antioxidant and ROS.

### Experimental Example 2. Kidney tissue fibrosis inhibitory effect in unilateral ischemia-reperfusion injury (UIRI) models

### 2-1. Preparation of animal model

7-week-old male mice (C57BL/6 mice) were anesthetized by intraperitoneal administration of Zoletil (30 mg/kg) and Rompun (10 mg/kg), and the left flank was incised to expose the pedicle of the left kidney, and blood vessels were ligated with a microvascular clamp, and then reperfusion was performed 26 minutes after the ligation, thereby obtaining progressive UIRI mouse models. The mice were treated with the graphene quantum dots according to Example 1 by caudal vein injection at a dose of 20 mg/kg one day before surgery and on days 3, 7, 14, 21, and 28 days after surgery, and 6 weeks after the procedure, the mice were sacrificed to extract kidneys, followed by observation of the induction of renal fibrosis.

The mice were classified into groups: Sham: normal control/vehicle administration (n=2); GQD: normal control/GQD administration (n=2); Unilateral IRI: Unilateral IRI experimental group/vehicle administration (n=4); Unilateral IRI + GQD: Unilateral IRI experimental group/GQD administration (n=4), and a control was administered a vehicle.

### 2-2. Analysis of changes in kidney size and body weight

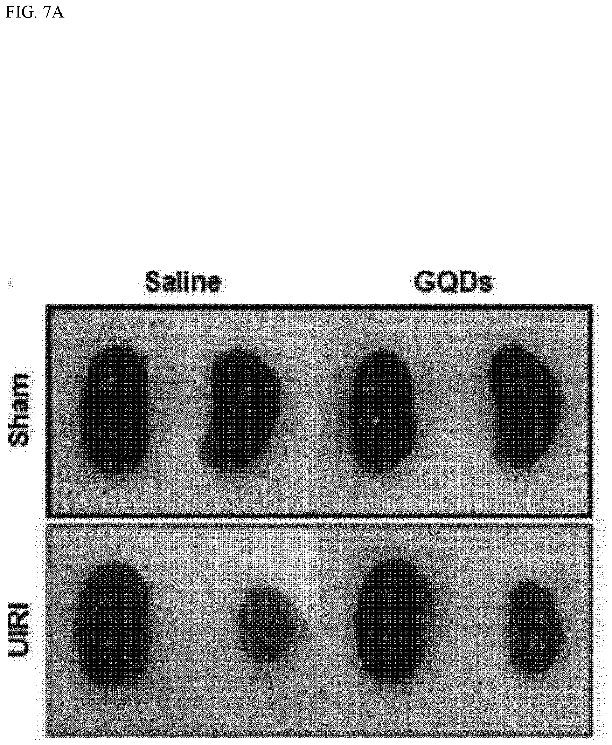

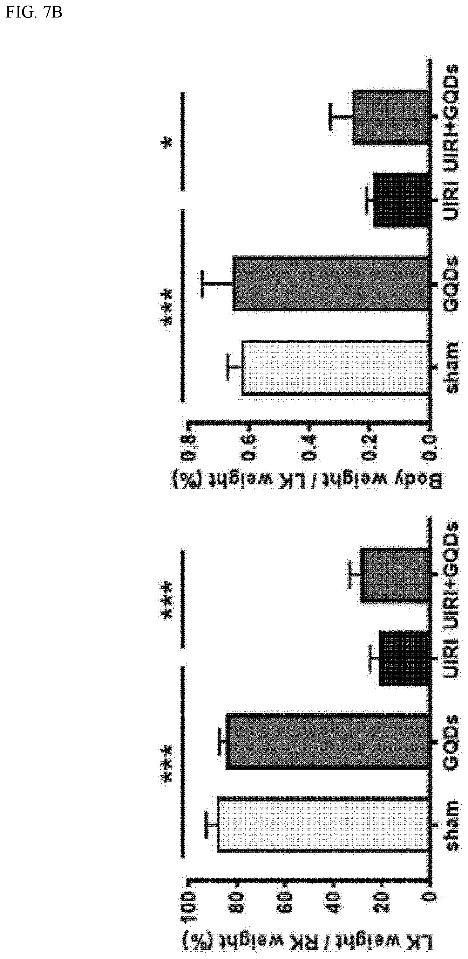

A decrease in kidney size and weight is a representative characteristic of UIRI induction, and as the experimental result, it was confirmed that the left kidney was significantly atrophied in the UIRI experimental group, whereas in the graphene quantum dot-administered group, the atrophy was reduced and the kidney weight was higher than that of the UIRI experimental group (FIGS. 7A and 7B).

### 2-3. Analysis of tubular atrophy inhibitory effect

To confirm the difference in the degree of renal fibrosis between a control and an experimental group, histological changes in the tubules were observed by periodic acid-Schiff staining.

The experimental method was performed in the same manner as in Experimental Example 1-2.

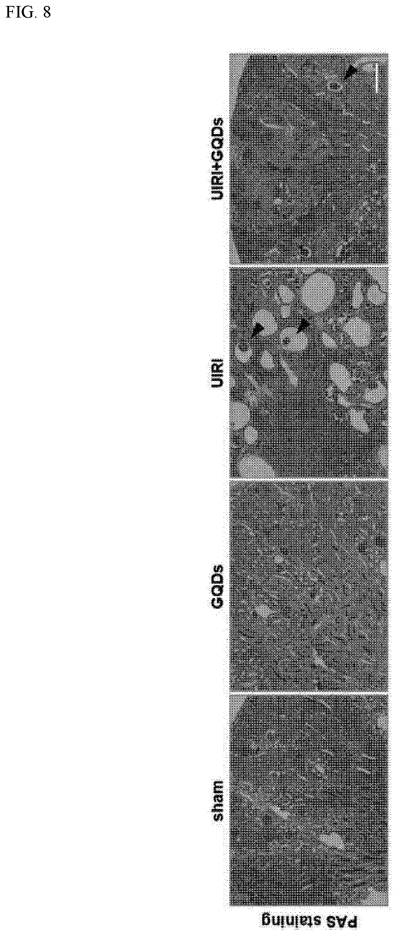

As a result, it was confirmed through PAS staining that clear tubular and glomerular atrophy was observed in the UIRI experimental group, but damage was prevented in the UIRI + GQD group (FIG. 8).

### 2-4. Analysis of collagen deposition inhibitory effect

To confirm the degree of renal fibrosis between a control and an experimental group, the degree of collagen deposition was confirmed through Masson's trichrome (MT) staining.

Kidney tissue samples were prepared in the same manner as in Experimental Example 1-2, and MT staining was performed according to the method of a trichrome stain kit (Modified Masson's).

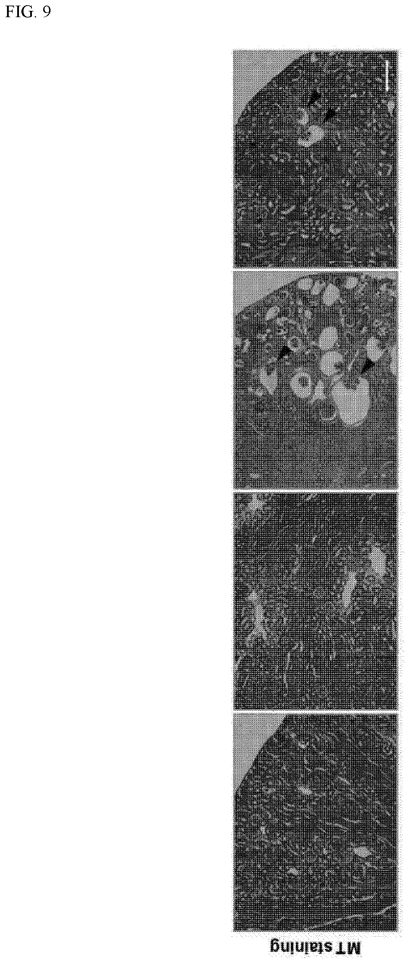

As a result, it was confirmed that excessive collage deposition was observed in the UIRI experimental group, but collagen deposition was reduced in the UIRI + GQD group (FIG. 9).

### 2-5. Analysis of change in TGF-β1 expression

To analyze the change in profibrotic factor, TGF-β1, expression in a control and an experimental group, immunohistochemistry (IHC) was performed.

Immunohistochemistry was performed in the same manner as in Experimental Example 1-3, and TGF-β1 (Abcam, ab92486) was used as a primary antibody.

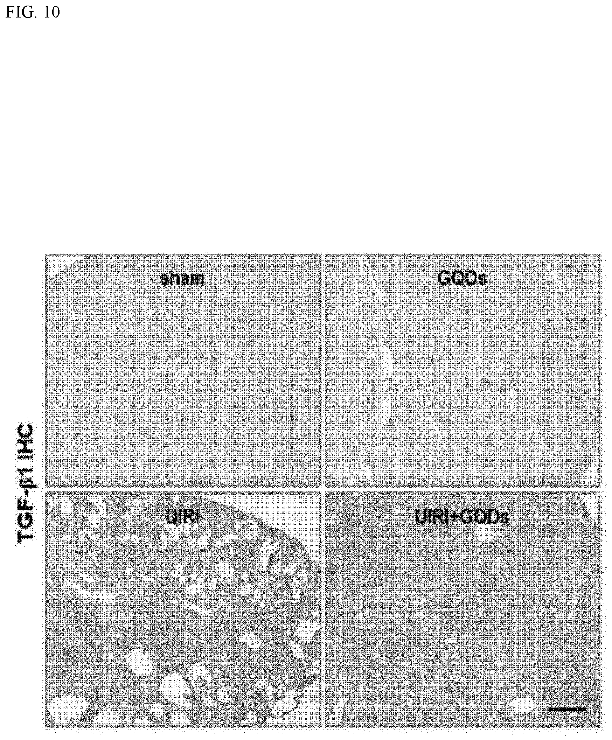

As a result, compared to the UIRI experimental group, in the UIRI + GQD group, it was confirmed that the TGF-β1 expression was reduced (FIG. 10).

### 2-6. Analysis of change in renal fibrosis-related protein expression

To analyze the change in related protein expression during UIRI-induced renal fibrosis, western blotting was performed.

Western blotting was performed in the same manner as in Experimental Example 1-4, and FN (Santa Cruz, sc-8422), Col 1a1 (Abcam, ab21286), α-SMA (Abcam, ab5694), vimentin (Cell Signaling Technology, 5741), E-cad (Abcam, ab11512), TGF-β1 (Abcam, ab92486), Smad7 (Abcam, ab216428) and GAPDH (Cell Signaling Technology, 2118) were used as primary antibodies.

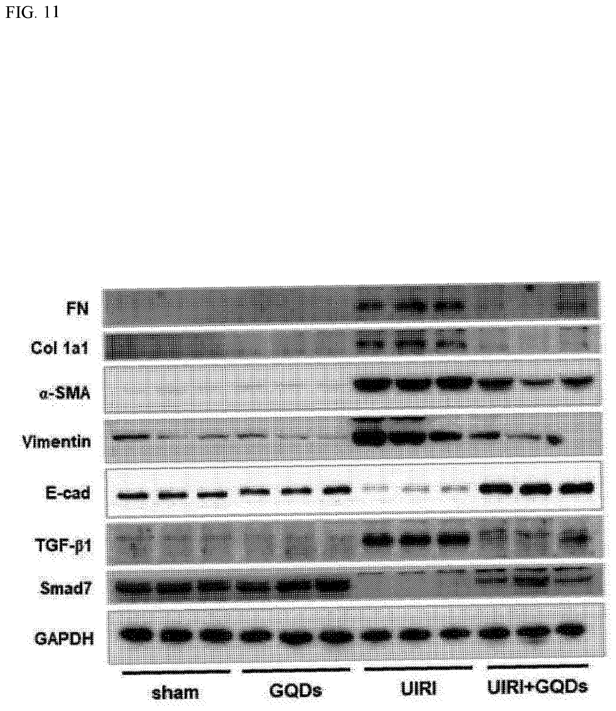

As a result, overexpressed protein levels of FN, Col 1a1, α-SMA, vimentin (EMT marker) and TGF-β1 in the URI group were significantly reduced by graphene quantum dots. In contrast, in the UIRI + GQD group, E-cad recovery indicates EMT inhibition, and an increased level of Smad7 indicates the regulation of TGF-β1/Smad signaling. Therefore, it was confirmed that graphene quantum dots have an effect of inhibiting renal fibrosis induced by UIRI (FIG. 11).

### Experimental Example 3. Effect of improving recombinant TGF-β-induced fibrosis in human renal proximal tubular epithelial cells

### 3-1. Cell culture

After subculture of primary cultured cells of human renal proximal tubular epithelial cells (hPTECs), 2 ng/ml of recombinant TGF-β (R&D; Minneapolis, MN, USA) was added to prepare a hPTEC fibrosis model. Subsequently, the cells were cultured with 2 and 10 µg/ml of graphene quantum dots for 48 hours. The control was treated only with graphene quantum dots to observe whether the graphene quantum dots are toxic to the cells.

### 3-2. Analysis of morphological change in hPTECs

To confirm the effect of graphene quantum dots on inhibition of the fibrosis of recombinant TGF-β-induced hPTECs, the morphological change in cells was observed under a microscope (Magnification XI00. Scale bar 200 µm).

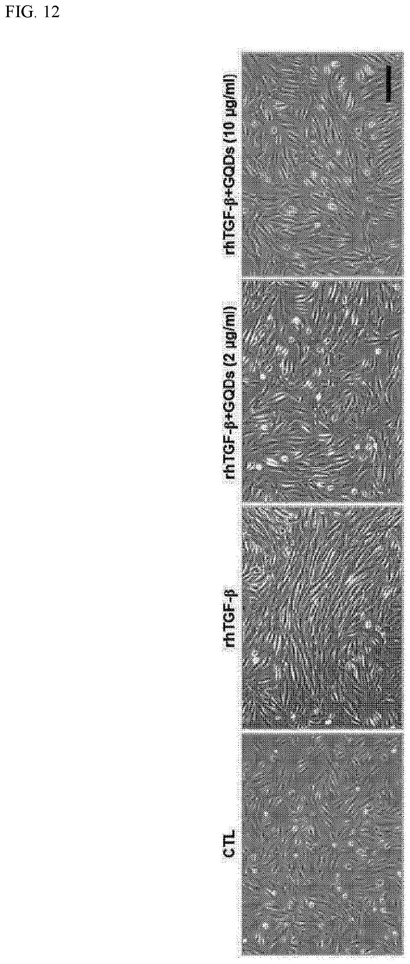

As a result, in the recombinant TGF-β-induced cells, due to the progression of EMT, it was confirmed that epithelial cells were transformed into myofibroblasts, and changed to an elongated shape such as a spindle shape. However, the morphology was maintained in the presence of graphene quantum dots, which can be seen to imply EMT inhibition (FIG. 12).

### 3-3. Analysis of change in expression of renal fibrosis-related protein

To confirm the change in the expression of renal fibrosis-related proteins between a control and an experimental group, immunofluorescence (IF) assay and qRT-PCR were performed.

For IF assay, cells were fixed with 4% paraformaldehyde at room temperature for 10 minutes, and incubated with primary antibodies Col 1a1 (Abcam, ab21286) and FN (Santa Cruz, sc-8422) in a 1% BSA blocking buffer at 4 °C overnight, and conjugated with donkey-anti-mouse Alexa Fluor488- and donkey-anti-goat Alexa Fluor555-binding secondary antibodies (both, Life Technologies; Waltham, MA, USA). High-quality fluorescence images were obtained through confocal microscopy (Leica TCS SP8 STED CW; Wetzlar, Germany).

qRT-PCR was performed in the same manner as in Experimental Example 1-4, and primer sequences are shown in Table 5 below.

**[Table 5]**

| **Primer** | **Sequence** | **Species** | **SEQ ID NO:** |
|---|---|---|---|
| Fibronectin (FN) Forward | CCA CCC CCA TAA GGC ATA GG | human | 15 |
| Fibronectin (FN) Reverse | GTA GGG GTC AAA GCA CGA GTC ATC | human | 16 |
| Collagen 1a1(Col 1a1) Forward | AGG GCT CCA ACG AGA TCG AGA TCC G | human | 17 |
| Collagen 1a1(Col 1a1) Reverse | TAC AGG AAG CAG ACA GGG CCA ACG | human | 18 |
| E-cadherin (E-cad) Forward | CAA TGC CGC CAT CGC TTA C | human | 19 |
| E-cadherin (E-cad) Reverse | ATG ACT CCT GTG TTC CTG TTA ATG | human | 20 |

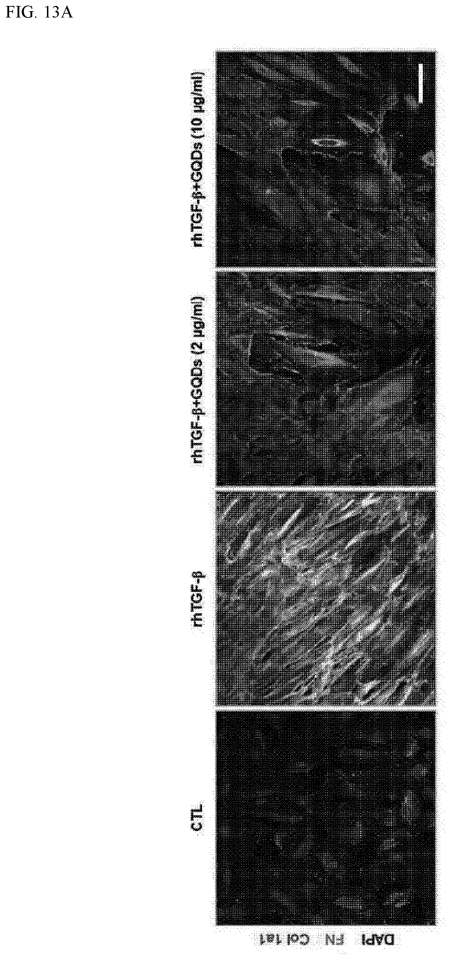

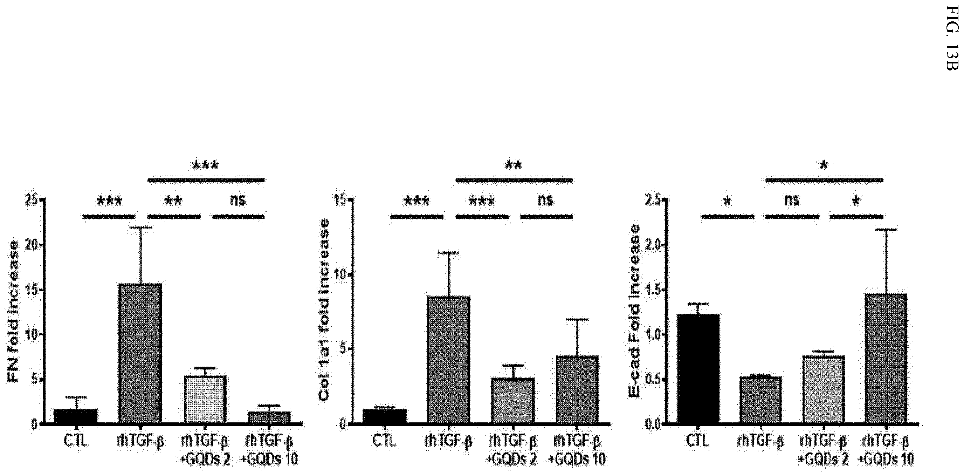

As the experimental result, it was confirmed that, as shown in IF images, FN and Col 1a1 overexpression caused by recombinant TGF-β was reduced by graphene quantum dots (FIG. 13A). It was confirmed that the mRNA levels of FN and Col 1a1 were also reduced in the graphene quantum dot-treated group. Moreover, the mRNA expression level of E-cadherin was increased by graphene quantum dots, suggesting EMT inhibition (FIG. 13B).

### 3-4. Analysis of apoptosis inhibitory effect

To confirm the apoptosis inhibitory effect of graphene quantum dots in recombinant TGF-β-induced hPTECs, annexin V/7-amino-actinomycin D (7-AAD) flow cytometry was performed.

Single cells (1 × 10⁶) of cultured hPTECs were resuspended in binding buffer, and incubated with 7-AAD (FITC Annexin V Apoptosis Detection Kit, BD Biosciences; San Jose, CA, USA) for 30 minutes at room temperature. After staining, the cells were analyzed using the BD FACS Canto platform (BD Biosciences; San Jose, CA, 35 USA).

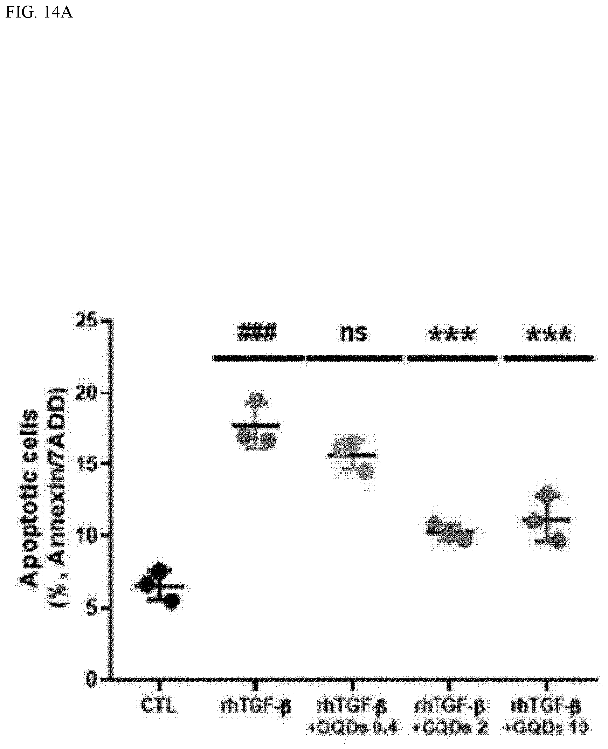

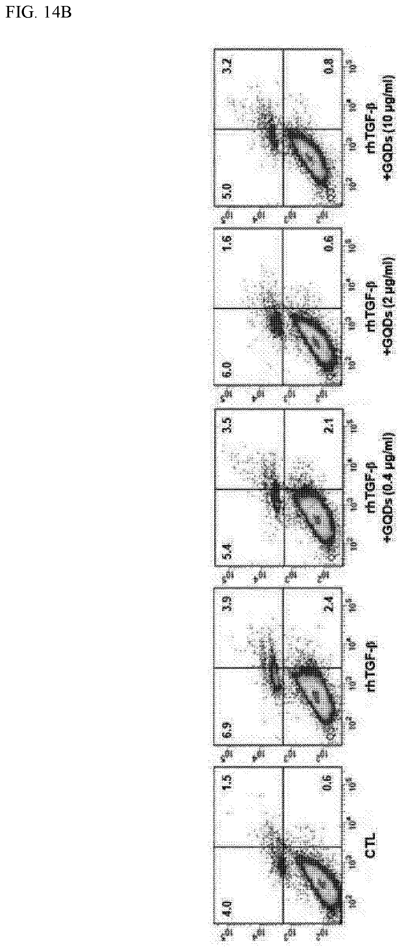

As a result, it was confirmed that the number of apoptotic cells was significantly increased in the recombinant TGF-β-induced hPTECs, but significantly decreased in the graphene quantum dot-treated group (FIGS. 14A and 14B).

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not restrictive in any aspect.

### [Industrial Applicability]

A pharmaceutical composition according to the present invention, which includes graphene quantum dots as an active ingredient, can inhibit renal fibrosis by decreasing the expression of renal fibrosis-related proteins, such as fibronectin, collagen 1a1, Bax, TGF-β1, Smad2/3 and α-SMA, and increasing the expression of E-cadherin, Smad7, MnSOD, and SOD1, and thus is expected to be effectively used in the prevention and treatment of a chronic kidney disease caused by renal fibrosis.

## Claims

1. A pharmaceutical composition for preventing or treating a chronic kidney disease, comprising:
graphene quantum dots as an active ingredient.

2. The composition of claim 1, wherein the chronic kidney disease is induced by renal fibrosis.

3. The composition of claim 1, wherein the chronic kidney disease is one or more selected from the group consisting of end-stage kidney disease (ESKD), diabetic nephropathy (DN), IgA nephropathy (IgAN), HIV-associated nephropathy, non-diabetic chronic kidney disease, focal segmental glomerulosclerosis (FSGS), minimal change disease (MCD), and xanthine oxidase deficiency.

4. The composition of claim 1, which decreases the expression of one or more proteins selected from the group consisting of fibronectin, collagen 1a1, Bcl-2-associated X protein (Bax), transforming growth factor-β1 (TGF-β1), Smad2/3, and α-smooth muscle actin (a-SMA).

5. The composition of claim 1, which increases the expression of one or more proteins selected from the group consisting of E-cadherin, Smad7, MnSOD, and SOD1.

6. The composition of claim 1, wherein the graphene quantum dots have an average lateral size of 1 to 5 nm.

7. The composition of claim 1, wherein the graphene quantum dots have an average height of 0.5 to 2.5 nm.

8. A method of treating a chronic kidney disease, comprising:
administering the pharmaceutical composition comprising graphene quantum dots as an active ingredient according to any one of claims 1 to 7 into a subject.

9. A use of the pharmaceutical composition comprising graphene quantum dots as an active ingredient according to any one of claims 1 to 7 for treating a chronic kidney disease.

10. A use of graphene quantum dots for preparing a drug used in the treatment of a chronic kidney disease.
